# EUROPÄISCHE PATENTSCHRIFT

(11) **EP 3 285 693 B1**
(45) Veröffentlichungstag und Bekanntmachung des Hinweises auf die Patenterteilung: **07.08.2019**
(21) Anmeldenummer: 16716598.4
(22) Anmeldetag: 15.04.2016
(51) Int. Cl.: A61F 2/50, A61F 2/68

(54) **VERFAHREN ZUR STEUERUNG EINES KÜNSTLICHEN KNIEGELENKES**
METHOD FOR CONTROLLING AN ARTIFICIAL KNEE JOINT
PROCÉDÉ POUR CONTRÔLER UNE PROTHÈSE DU GENOU

(30) Priorität: 24.04.2015 DE 102015106389
(43) Veröffentlichungstag der Anmeldung: 28.02.2018
(73) Patentinhaber: Otto Bock Healthcare Products GmbH, 1070 Wien (AT)
(72) Erfinder: SEIFERT, Dirk, 1070 Wien (AT)
(74) Vertreter: Lins, Martina
(86) Internationale Anmeldenummer: PCT/EP2016/058406
(87) Internationale Veröffentlichungsnummer: WO 2016/169855

(56) Entgegenhaltungen:
- WO-A1-2014/079588
- US-A1- 2008 114 272
- US-A1- 2009 192 625
- US-B1- 6 755 870

## Beschreibung

Die Erfindung betrifft ein Verfahren zur Steuerung eines künstlichen Kniegelenkes, insbesondere eines Prothesenkniegelenkes, mit einem Oberteil und einem Unterteil, die schwenkbar um eine Schwenkachse aneinander befestigt sind, und einer Widerstandseinheit, die zwischen dem Oberteil und dem Unterteil angeordnet ist und eine Verstelleinrichtung aufweist, über die der Dämpfungswiderstand verändert werden kann, mit einer Steuereinheit, die mit der Verstelleinrichtung gekoppelt ist und die zumindest mit einem Sensor verbunden ist, wobei die Verstellung auf der Grundlage von Sensordaten erfolgt. Das Verfahren ist ebenfalls zur Steuerung von Orthesenkniegelenken oder Exoskelettkniegelenken einsetzbar und dafür vorgesehen.

Kniegelenke für Orthesen, Exoskelette oder Prothesen weisen ein Oberteil mit einem oberen Anschlussteil und einem Unterteil mit einem unteren Anschlussteil auf, die gelenkig miteinander verbunden sind. An dem oberen Anschlussteil sind in der Regel Aufnahmen für einen Oberschenkelstumpf oder eine Oberschenkelschiene angeordnet, während an dem unteren Anschlussteil ein Unterschenkelschaft oder eine Unterschenkelschiene angeordnet sind. Im einfachsten Fall sind das Oberteil und das Unterteil durch ein einachsiges Gelenk verschwenkbar miteinander verbunden.

Um unterschiedliche Anforderungen während der verschiedenen Phasen eines Schrittes oder bei anderen Bewegungen oder Verrichtungen möglichst natürlich darzustellen oder zu unterstützen, ist häufig eine Widerstandseinrichtung vorgesehen, die einen Flexionswiderstand und einen Extensionswiderstand bereitstellt. Über den Flexionswiderstand wird eingestellt, wie leicht sich das Unterteil im Verhältnis zum Oberteil bei einer aufgebrachten Kraft nach hinten schwingen lässt. Der Extensionswiderstand bremst die Vorwärtsbewegung des Unterteils und bildet unter anderem einen Streckanschlag aus.

Aus der DE 10 2008 008 284 A1 ist ein orthopädietechnisches Kniegelenk mit einem Oberteil und einem verschwenkbar daran angeordneten Unterteil bekannt, dem mehrere Sensoren zugeordnet sind, beispielsweise ein Beugewinkelsensor, ein Beschleunigungssenor, ein Neigungssenor und/oder ein Kraftsensor. In Abhängigkeit von den Sensordaten wird die Position des Extensionsanschlages ermittelt.

Die DE 10 2006 021 802 A1 beschreibt eine Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß. Die Anpassung erfolgt an das Treppaufgehen, wobei ein momentenarmes Anheben des Prothesenfußes detektiert und die Flexionsdämpfung in einer Anhebephase auf unterhalb eines Niveaus, das für ein Gehen in der Ebene geeignet ist, abgesenkt wird. Die Flexionsdämpfung kann in Abhängigkeit von der Veränderung des Kniewinkels und in Abhängigkeit von der auf den Unterschenkel wirkenden Axialkraft angehoben werden.

Die DE 10 2009 052 887 A1 beschreibt unter anderem ein Verfahren zur Steuerung eines orthetischen oder prothetischen Gelenkes mit einer Widerstandseinrichtung und Sensoren, wobei über Sensoren während der Benutzung des Gelenkes Zustandsinformationen bereitgestellt werden. Die Sensoren erfassen Momente oder Kräfte, wobei die Sensordaten von zumindest zwei der ermittelten Größen durch eine mathematische Operation miteinander verknüpft werden und dadurch eine Hilfsvariable errechnet wird, die der Steuerung des Beuge- und/oder Streckwiderstandes zugrunde gelegt wird.

Zur Steuerung der Veränderung des Dämpfungsverhaltens werden gemäß dem Stand der Technik die Sensordaten quantitativ ausgewertet, das heißt, dass in der Regel bestimmte Grenzwerte vorgegeben werden, bei deren Erreichen oder Nichterreichen der Aktuator aktiviert oder deaktiviert wird, so dass die Widerstandseinrichtung einen erhöhten oder verringerten Flexions-oder Extensionswiderstand bereitstellt.

Die US 2008-114272 A1 beschreibt ein Verfahren zur Steuerung eines Prothesenkniegelenkes, bei dem Geschwindigkeitsdaten bei verschiedenen Bewegungsgeschwindigkeiten des Prothesenträgers gemessen werden. Die Daten werden korrespondierend zu der Bewegungsgeschwindigkeit gespeichert, und die Dämpfung wird iterativ verändert, um einen vorbestimmten oder errechneten Zielwert zu erreichen, bis die Dämpfung innerhalb des Speicherraums konvergiert. Die konvergierten Dämpfungswerte werden verwendet, um durch Veränderung der Viskosität eines magnetorheologischen Fluids die Dämpfung in dem Prothesenkniegelenk zu steuern. Werden in der Flexionsphase während der Schwungphase zu große Kniewinkel detektiert, wird die Dämpfung erhöht. Liegt der Impuls des Extensionsanschlages innerhalb eines akzeptablen Bereiches, wird die Extensionsdämpfung unverändert gelassen. Sobald sich Änderungen an den gemessenen Werten im Vergleich zu den Zielwerten ergeben, wird nachjustiert. Als Basisgröße wird die Gehgeschwindigkeit eingesetzt, die über die Kontaktdauer oder Axialkraftdauer bestimmt wird. Je schneller die Gehgeschwindigkeit, desto geringer ist die Verweildauer des Fußes auf dem Boden. Durch die Modulation des Dämpfungsniveaus in einem Zeitfenster kann die Änderung beispielsweise über einen festgelegten oder vorbestimmten Kniewinkelbereich erfolgen.

In der US 6 755 870 B1 ist ein Verfahren zum Steuern eines Dämpfungsverhaltens eines prothetischen Kniegelenks beschrieben, bei dem für Sondersituationen besondere Dämpfungsalgorithmen eingeleitet werden. Als Sondersituation werden Treppensteigen, Stolpern und Stürzen angesehen. In Abhängigkeit von Messdaten werden durch die Steuereinheit Steuersignale für eine Kolben-Zylinder-Einrichtung erzeugt und ein Elektromagnet angesteuert, um ein definiertes Magnetfeld zu erzeugen. Dadurch wird eine bestimmte Viskositätsänderung einer magnetorheologischen Flüssigkeit hervorgerufen. Da dies innerhalb einer sehr kurzen Zeitspanne von 3 ms bis 5 ms erfolgen kann, ist ein Einsatz der Dämpfung als Rückfallbremse möglih. Wenn der Träger der Beinprothese stolpert, kann durch unmittelbar aufbauende Dämpfung ein Einklappen des Unterschenkelteils frühzeitig verhindert werden.

Aufgabe der vorliegenden Erfindung ist es, ein Verfahren bereitzustellen, bei dem Problemsituationen beim Gehen frühzeitig erkannt werden und durch geeignete Anpassungen des Widerstandes schnell darauf reagiert werden kann.

Erfindungsgemäß wird diese Aufgabe durch ein Verfahren mit den Merkmalen des Hauptanspruches gelöst. Vorteilhafte Ausgestaltungen und Weiterbildungen der Erfindung sind in den Unteransprüchen sowie der Beschreibung und den Figuren offenbart.

Das Verfahren zur Steuerung eines künstlichen Kniegelenkes mit einem Oberteil und einem Unterteil, die schwenkbar um eine Schwenkachse aneinander befestigt sind, und einer Widerstandseinheit, die zwischen dem Oberteil und dem Unterteil angeordnet ist und eine Verstelleinrichtung aufweist, über die der Dämpfungswiderstand verändert werden kann, und einer Steuereinheit, die mit der Verstelleinrichtung gekoppelt ist und die zumindest mit einem Sensor verbunden ist, wobei die Verstellung auf der Grundlage von Sensordaten erfolgt, sieht vor, dass während der Schwungphase zumindest eine Kenngröße aus einer Gruppe enthaltend den Kniewinkel, die Kniewinkelgeschwindigkeit, die Kniewinkelbeschleunigung, den Unterschenkelwinkel, die Unterschenkelgeschwindigkeit, die Unterschenkelbeschleunigung, das Knöchelmoment und die Axialbelastung erfasst wird, der Verlauf der Kenngröße bestimmt und der Dämpfungswiderstand verändert wird, wenn nach Erreichen eines Extremwertes der Kenngröße sich das Monotonieverhalten des Verlaufes der Kenngröße innerhalb der Schwungphase ändert, beispielsweise wenn nach Erreichen eines Extremwertes der Kenngröße der Verlauf der Kenngröße innerhalb der Schwungphase wieder ansteigt. Das erfindungsgemäße Verfahren ist insbesondere zur Erkennung von Abweichungen innerhalb eines üblichen Bewegungsablaufes geeignet, insbesondere zur Erkennung des Abweichens von einem Gehen in der Ebene oder auf Rampen oder anderen periodisch wiederkehrenden, gleichförmigen Bewegungsmustern, die vorzugsweise wenigstens abschnittsweise ein charakteristisches Monotonieverhalten aufweisen. Hierzu werden der Unterschenkel- und/oder Kniewinkelverlauf, deren erste und zweite Ableitung nach der Zeit sowie gegebenenfalls die Axialbelastung auf das Unterteil und gegebenenfalls das Knöchelmoment überwacht. Wird während der Schwungphase, die beispielsweise durch die Abwesenheit eines Knöchelmomentes oder einer auf das Unterteil wirkenden Axialkraft entlang des Unterschenkelrohres oder eine Unterschenkelschiene erkannt werden kann, ein von dem üblichen Verlauf der Kenngrößen abweichender Kenngrößenverlauf nach Erreichen eines Extremwertes, beispielsweise eines lokalen Maximums oder eines lokalen Minimums, erkannt, ist vorgesehen, dass der Dämpfungswiderstand verändert wird, so dass das Abweichen von bekannten Bewegungsmustern als Auslösesignal dafür genommen wird, dass der Dämpfungswiderstand verändert wird, beispielsweise indem der Dämpfungswiderstand vergrößert oder verkleinert wird. Die Änderung des Monotonieverhaltens ist der Indikator dafür, dass ein von dem üblichen Gangmuster abweichendes Verhalten auftritt, z.B. eine Störung durch ein Hindernis. Dabei kann die Änderung des Monotonieverhaltens sowohl in einem Absinken als auch in einem Ansteigen des Kenngrößenverlaufs bestehen. Wird nach einem Extremwert ein bestimmter Kenngrößenverlauf für eine bestimmte Dauer oder einen bestimmten Winkelbereich erwartet und tritt innerhalb dieses Zeit- oder Positionsbereiches eine Monotonieveränderung des Kenngrößenverlaufes auf, wird dies als Auslöser für die Veränderung des Dämpfungswiderstandes verwendet. Das Verfahren ist zur Steuerung sowohl von Prothesen als auch von Orthesen und Exoskeletten vorgesehen. Wird nachfolgend von Orthesen gesprochen, so gelten die Ausführungen ebenso für die Sonderform der Orthese in Gestalt eines Exoskelettes.

Das Abweichen von bestimmten Kenngrößenverläufen nach Erreichen eines Extremwertes, insbesondere eines lokalen Extremwertes, hat eine besondere Gehsituation oder einen besonderen Umstand während der Schwungphase zur Grundlage, insbesondere kann davon ausgegangen werden, dass der Fuß, sei es der Prothesenfuß oder der natürliche Fuß im Falle einer Orthese, gegen ein Hindernis stößt, so dass die Gefahr eines Stolperns gegeben ist. In einer solchen Situation kann es vorteilhaft sein, dass das künstliche Kniegelenk gegen ein starkes Einbeugen gesichert wird, um den Nutzer des künstlichen Kniegelenkes in die Lage zu versetzen, das künstliche Kniegelenk zum Abfangen der Bewegung und des Körpergewichtes einzusetzen, so dass der Dämpfungswiderstand gegen eine Flexion, also gegen ein Einbeugen, erhöht wird.

Vorteilhafterweise wird der Dämpfungswiderstand auf ein Niveau der Standphasendämpfung oder darüber hinaus erhöht. Das Niveau der Standphasendämpfung wird für den jeweiligen Patienten, dessen Gewicht sowie dessen Aktivitätsniveau eingestellt und somit individuell angepasst. In der Standphase wird ein Flexions-Dämpfungs-Niveau bereitgestellt, das größer als das während einer normalen Schwungphase ist, da während der Schwungphase ein Einbeugen des künstlichen Kniegelenkes erwünscht wird, um ein Durchschwingen des Fußes oder Prothesenfußes nach vorne zum Aufsetzen der Ferse zu ermöglichen. Der Dämpfungswiderstand bei Erkennen eines besonderen Gangmusters somit auf ein Niveau der Standphasendämpfung oder darüber hinaus erhöht, um ein Einbeugen zu vermeiden oder im Vergleich zur Schwungphase zu erschweren.

Vorteilhafterweise wird als Extremwert für den Unterschenkelwinkel, die Unterschenkelgeschwindigkeit, die Unterschenkelbeschleunigung, den Kniewinkel, die Kniewinkelgeschwindigkeit oder die Kniewinkelbeschleunigung ein Maximum verwendet. Der Kniewinkelverlauf für das normale Gehen sowie das Gehen entlang von Rampen weist einen glockenförmigen oder angenähert glockenförmigen Verlauf mit einem lokalen Maximum auf. Die Kniewinkelgeschwindigkeit weist einen angenähert sinusförmigen Verlauf mit einem lokalen Maximum zu Beginn der Schwungphase und einem lokalen Minimum am Ende der Schwungphase auf. Die Kniewinkelbeschleunigung weist einen leicht phasenversetzten Verlauf ähnlich dem der Kniewinkelgeschwindigkeit auf. Die Belastung auf das Unterteil, sei es durch ein Knöchelmoment oder eine Axialkraft sinkt auf 0, nachdem eine Zehenablösung, das sogenannte "toe off", stattgefunden hat. Eine externe Axialkraft wird nach Abheben des Fußes nicht mehr aufgebracht, ebenso fehlt ein Knöchelmoment. Die lokalen maximalen Kenngrößenverläufe liegen zeitlich hintereinander und im Bereich der Schwungphasenflexion, zumindest für die Kniewinkelgeschwindigkeit und den Kniewinkel. Die Kniewinkelbeschleunigung hat ihren Maximalwert im Bereich der initialen Schwungphase oder am Ende der sogenannten Vorschwungphase (pre-swing phase), so dass bei Berücksichtigung aller Kenngrößen, die sich aus dem Kniewinkel, ggf. zusammen mit dem Knöchelmoment und der Axialkraft, ableiten lassen, eine sichere Überwachung des Gangverhaltens sowie eine Erkennung einer Stolpergefahr durch das Verfahren bereitgestellt werden kann.

Auch der Unterschenkelwinkel, der der Winkel des Unterschenkels zu der Vertikalen ist, sowie die daraus abgeleiteten Kenngrößen der Unterschenkelgeschwindigkeit und der Unterschenkelbeschleunigung, weist einen sinusartigen Verlauf mit einem Extremwert bei Erreichen des maximalen Kniewinkels und einem entgegen gerichteten Extremwert beim Fersenkontakt oder Heel Strike. Bei Störungen des Verlaufes einer oder mehrerer Kenngrößen, die mit dem Unterschenkel oder der Unterschenkelstellung im Raum zusammenhängen, wird der Dämpfungswiderstand verändert. Die Stellung des Unterschenkels und damit der Unterschenkelwinkel kann über einen Inertialwinkelsensor erfasst werden, die Winkelgeschwindigkeit oder -beschleunigung kann über die Ableitung nach der Zeit errechnet werden.

Neben einer häufig benötigten Erhöhung des Flexionswiderstandes, um eine Belastung des Gelenkes zu ermöglichen, kann auch der umgekehrte Weg eine sinnvolle Möglichkeit sein, um die Sicherheit des Patienten zu erhöhen. Dazu wird der Dämpfungswiderstand gegen eine Flexion in der Schwungphase auf ein Niveau unterhalb eines normalen Schwungphasenwiderstandes abgesenkt, wenn das Knöchelmoment oder die Axialkraft, also die Belastung auf das Unterteil, einen Schwellwert unterschreiten oder Null sind und der Kniewinkel, die Kniewinkelgeschwindigkeit und/oder die Kniewinkelbeschleunigung nach Erreichen eines Extremwertes eine Monotonieveränderung erfährt, z.B. nach Erreichen eines Maximums und einer kurzen Phase des Absinkens wieder ansteigt oder nach Erreichen eines Minimums und einer kurzen Phase des Ansteigens wieder abfällt. Der Schwellwert für die Detektion der Belastung auf das Unterteil liegt nah bei einer vollständigen Entlastung. Eine solche "Elevating"-Strategie ist dann sinnvoll, wenn ein weiteres Durchschwingen des Unterteils und damit eine Erhöhung des Abstandes des Fußes von dem Boden notwendig sind.

Für die Kenngrößen und insbesondere das Ansteigen des Kenngrößenverlaufes über einen erwarteten Wert nach Erreichen eines Extremwertes wird vorteilhafterweise ein Schwellwert a festgelegt, der überschritten werden muss, damit der Dämpfungswiderstand verändert wird. Erst nach Erreichen des Schwellwertes oder Überschreiten des Schwellwertes erfolgt ein Eingriff in den üblichen, voreingestellten Widerstandsverlauf, um zu vermeiden, dass bei geringfügigen Abweichungen in dem Bewegungsablauf bereits ungewollt Dämpfungsänderungen vorgenommen werden. Dadurch wird die Toleranz von Bewegungsabweichungen sowie von Signalrauschen erhöht, ohne dass die Sicherheit des Patienten gefährdet wird.

Weiterhin ist es möglich, einen Zeitschwellwert T festzulegen, der nach Erreichen des Extremwertes überschritten werden muss, damit der Dämpfungswiderstand verändert wird. Dadurch wird sichergestellt, dass tatsächlich ein Extremwert vorliegt und nicht nur ein geringfügiges Abweichen des Kenngrößenverlaufes von dem üblichen Verlauf.

Der Verlauf der Kenngrößen wird permanent mit einer hohen Abtastrate überwacht, die Extremwerte der Kenngrößen werden vorteilhafterweise in Echtzeit ermittelt, um Störungen im Bewegungsablauf frühzeitig zu erkennen und sehr früh mit den Korrekturmaßnahmen hinsichtlich des Dämpfungswiderstandes reagieren zu können. Veränderungen des Dämpfungswiderstandes aufgrund des oben beschriebenen Korrekturmechanismusses können registriert und in einem Speicher abgelegt werden, so dass eine statistische Auswertung der Eingriffe und Korrekturen möglich ist. Dies erlaubt einen statistischen Nachweis über die Funktionsweise und die Nützlichkeit des eingesetzten Verfahrens.

Der Vorteil des Verfahrens ist insbesondere darin zu sehen, dass es unabhängig von individuellen Einstellungen für den Patienten arbeiten kann. Es werden nur relative Beziehungen zwischen den jeweiligen Kenngrößenverläufen ermittelt, das Gewicht, die Ganggeschwindigkeit oder ähnliches sind irrelevant für die Detektion von Gangmusterabweichungen, beispielsweise das Stolpern. Es wird lediglich ein intrinsisches Muster der Bewegung genutzt, was das Verfahren sicher, störungsunanfällig und vielseitig einsetzbar macht.

Das Verfahren ist insbesondere für langsam gehende Patienten geeignet, bei denen eine größere Gefahr des Hängenbleibens beim Gehen und dem damit verbundenen Stolpern besteht.

Eine Weiterbildung der Erfindung sieht vor, dass diejenigen Monotonieänderungen nicht berücksichtigt werden, die in einem ungestörten Schwungphasenverlauf auftreten, beispielsweise nach Erreichen von Extremwerten, insbesondere absoluten Extremwerten. In der Schwungphase können für eine Kenngröße mehrere Extremwerte auftreten, die jeweils eine Veränderung des Monotonieverhaltens des Kenngrößenverlaufs zur Folge haben. Die Änderungen des Monotonieverlaufes sollen nur dann für die Änderung des Dämpfungsverhaltens berücksichtigt werden, wenn sie nicht ohnehin im normalen Schwungphasenverlauf auftreten würden, also für den Knie- oder Unterschenkelverlauf zwischen dem absoluten Maximum und dem absoluten Minimum der Kenngrößenverläufe, Gleiches gilt auch für die jeweiligen ersten Ableitungen dieser Kenngrößen nach der Zeit.

Die Widerstandseinheit kann beispielsweise als Aktuator, beispielsweise als hydraulische, pneumatische, magnetorheologische, magnetische, elektrische, mechanische oder elektromagnetische Widerstandseinheit ausgestaltet sein. Bei hydraulischen oder pneumatischen Widerstandseinheiten werden Überströmkanäle geschlossen, so dass diese Überströmkanäle kein Medium mehr aus einer Extensionskammer in eine Flexionskammer strömen kann. Auf diese Weise kann der Fluss des Mediums zwischen der Extensionskammer und der Flexionskammer ggf. auch vollständig unterbunden werden. Bei mechanischen Widerstandseinrichtungen wird beispielsweise die Reibung soweit erhöht, dass keine weitere Flexion stattfinden kann. Gleiches gilt für elektrisch aktuierte Widerstandseinheiten.

Es können auch Aktuatoren zum Einsatz kommen, die sowohl Energie aktiv in das System einbringen, als auch umgekehrt dem System Energie entziehen und auf diese Weise als Widerstandseinheit wirken. Aktuatoren können beispielsweise als Elektromotoren, hydraulische oder pneumatische Pumpen oder piezoelektrische Elemente ausgebildet sein.

Nachfolgend wird ein Ausführungsbeispiel der Erfindung anhand der beigefügten Figuren näher erläutert. Es zeigen:
- Figur 1 -: eine schematische Darstellung einer Prothese,
- Figur 2 -: einen schematischen Verlauf der Kenngrößen; sowie
- Figur 3 -: eine schematische Darstellung einer Schwellwertberücksichtigung.

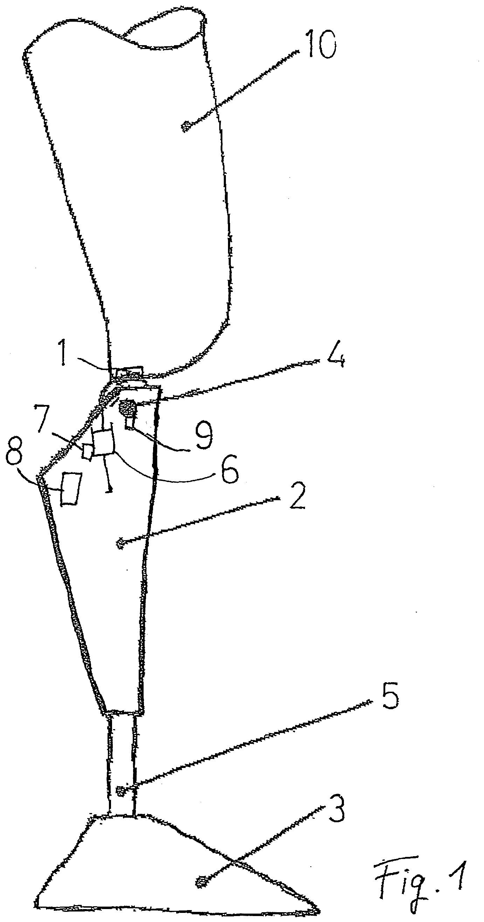

In der Figur 1 ist in einer schematischen Darstellung eine Beinprothese mit einem Oberteil 1 gezeigt, an dem ein Oberschenkelschaft 10 zur Aufnahme eines Oberschenkelstumpfes befestigt ist. An dem Oberteil 1 ist ein Unterteil 2 in Gestalt eines Unterschenkelteils schwenkbar angeordnet. Das Unterteil 2 ist an dem Oberteil 1 um eine Schwenkachse 4 schwenkbar gelagert. Das Unterteil 2 weist ein Unterschenkelrohr 5 auf, an dessen distalen Ende ein Prothesenfuß befestigt ist, in dem eine Einrichtung zur Ermittlung der wirksamen Axialkraft auf das Unterschenkelrohr 5 sowie des Knöchelmomentes, das um die Befestigungsstelle des Prothesenfußes 3 an dem Unterschenkelrohr 5 wirksam ist, untergebracht sein kann.

In oder an dem Unterteil 2 ist eine Widerstandseinrichtung 6, die beispielsweise als Dämpfer oder Aktuator ausgebildet sein kann, angeordnet, die sich zwischen dem Oberteil 1 und dem Unterteil 2 abstützt, um einen einstellbaren Extensionswiderstand und Flexionswiderstand bereitzustellen. Der Widerstandseinrichtung 6 ist einer Verstelleinrichtung 7 zugeordnet, beispielsweise ein Motor, ein Magnet oder ein anderer Aktuator, über den der jeweilige Widerstand innerhalb der Widerstandseinrichtung 6 verändert werden kann. Ist die Widerstandseinrichtung 6 als Hydraulikdämpfer ausgebildet, kann über die Verstelleinrichtung 7 der jeweilige Strömungsquerschnitt vergrößert oder verkleinert werden. Dies kann durch Öffnen oder Schließen von Ventilen oder Veränderungen von Viskositäten oder magnetorheologischer Eigenschaften geschehen. Ist die Widerstandseinrichtung als ein Elektromotor im Generatorbetrieb ausgebildet, kann durch Veränderung des elektrischen Widerstandes eine Vergrößerung oder Verringerung der jeweiligen Widerstände gegen eine Flexion oder Extension eingestellt werden.

Um die Verstelleinrichtung 7 aktivieren oder deaktivieren zu können, ist eine Steuerungseinrichtung 8 dem Unterteil 2 zugeordnet, insbesondere in einer Unterschenkelverkleidung angebracht, über die ein entsprechendes Aktivierungs- oder Deaktivierungssignal an die Verstelleinrichtung 7 abgegeben wird. Die Verstelleinrichtung 7 wird auf der Basis von Sensordaten aktiviert oder deaktiviert, die Sensordaten werden von einem oder mehreren Sensoren 9 geliefert, die an dem künstlichen Kniegelenk angeordnet sind. Dies können Winkelsensoren, Beschleunigungssensoren und/oder Kraftsensoren sein. Die Sensoren 9 sind mit der Steuereinrichtung 8 verbunden, beispielsweise per Kabel oder über eine drahtlose Sendeeinrichtung.

Über die Sensoren 9 wird der gesamte Schrittzyklus von dem Fersenstoß (Heel Strike) bis zum erneuten, nachfolgenden Heel Strike HS überwacht, somit auch die gesamte Schwungphase mit der Schwungphasenextension und der Schwungphasenflexion.

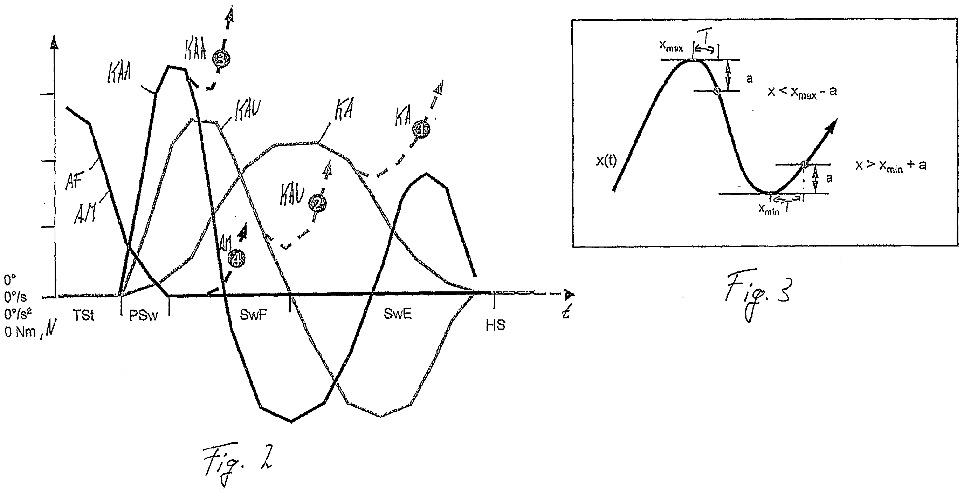

Zur Steuerung der Dämpfung über die Widerstandseinrichtung 6 wird insbesondere der Kniewinkelverlauf sowie dessen erste und zweite Ableitung nach der Zeit überwacht. Der Kniewinkel KA ist in der Figur 2 über die Zeit dargestellt. Ebenso ist der Verlauf der Kniewinkelgeschwindigkeit KAV und der Kniewinkelbeschleunigung KAA über die Zeit in der Figur 2 dargestellt. Darüber hinaus ist dem Diagramm die Belastung in Gestalt einer Axialkraft AF und eines Knöchelmomentes AM dargestellt, die sich im Wesentlichen in ihrem Verlauf entsprechen, so dass beide Belastungsgrößen AF, AM in einer Kurve dargestellt sind.

Im Bereich der terminalen Standphase TSt verringert sich die Axialbelastung AF ebenso wie das Knöchelmoment AM, nach Beendigung der terminalen Standphase TSt steigen der Kniewinkel KA, die Kniewinkelgeschwindigkeit KAV sowie die Kniewinkelbeschleunigung KAA an. Der Verlauf des Kniewinkels KA ist im Wesentlichen glockenförmig und steigt bis zum Ende der Schwungphasenflexion SwF bis zu einem Kniewinkelmaximum an, um dann im Wesentlichen ebenfalls glockenförmig in der Schwungphasenextensionsphase SwE bis zum Heel Strike HS sich zu verringern und beim Heel Strike HS in der maximal extendierten Stellung vorzuliegen, bei der der Kniewinkel KA als null Grad angenommen wird.

Die Kniewinkelgeschwindigkeit KAV weist einen im Wesentlichen sinusförmigen Verlauf auf. Zu Beginn der Schwungphase, in der sogenannten Pre-Swing-Phase PSw ist ein vergleichsweise schneller Anstieg der Kniewinkelgeschwindigkeit KAV festzustellen, nach Erreichen eine lokalen Maximums fällt die Kniewinkelgeschwindigkeit auf null bei Erreichen eines maximalen Kniewinkels KA ab, wird negativ, erreicht ein relatives Minimum und steigt dann bis zum Heel Strike HS wieder an, um bei Erreichen einer maximal extendierten Stellung null zu sein.

Die Kniewinkelbeschleunigung KAA erreicht ihr relatives Maximum am Ende der Pre-Swing-Phase PSw vor dem Maximum der Kniewinkelgeschwindigkeit KAV, hat bei Erreichen der maximalen Kniewinkelgeschwindigkeit KAV einen ersten Nulldurchgang, erreicht ein Minimum im Bereich des Nulldurchganges der Kniewinkelgeschwindigkeit KAV und ein zweites relatives Maximum am Ende der Schwungphasenextension.

Der so dargestellte Kniewinkelverlauf, Kniewinkelgeschwindigkeitsverlauf und Kniewinkelbeschleunigungsverlauf tritt jeweils auf, wenn ein ungestörtes Gehen erfolgt. Eine Störung, beispielsweise Stolpern, kann angenommen werden, wenn der Kniewinkel KA ein lokales Maximum erreicht hat und wieder unterschreitet und anschließend wieder ansteigt, was durch die gestrichelte Kurve KA₁ in der Figur 2 dargestellt ist.

Weiterhin kann eine Störung oder ein Stolpern angenommen werden, wenn nach Erreichen eines lokalen Maximums der Kniewinkelgeschwindigkeit KAV und einem Abfall der Kniewinkelgeschwindigkeit KAV dieses innerhalb der Schwungphase erneut wieder ansteigt, was durch den Kurvenverlauf KAV₂ dargestellt ist. Ebenfalls kann ein Stolpern angenommen werden, wenn sich die Kniewinkelbeschleunigung KAA nach Erreichen eines lokalen Maximums wieder verringert und anschließend wieder ansteigt, was durch den Kurvenverlauf KAA₃ dargestellt ist.

Die jeweiligen Maxima der Kurvenverläufe werden in Echtzeit bestimmt. Für das Unterschreiten der jeweiligen Maxima sowie den Anstieg des Kurvenverlaufes bzw. des Sensorsignals können Schwellwerte a definiert sein, um das Signalrauschen oder leichte Bewegungsabweichungen tolerieren zu können.

In der Figur 3 ist schematisch ein Kenngrößenverlauf dargestellt. Solange der Schwellwert a nicht überschritten wird, also eine Abweichung innerhalb eines bestimmten Zeitschwellwertes T nicht ausreichend groß ist, wird keine Veränderung der Dämpfungseinstellung vorgenommen. Überschreitet der Kenngrößenverlauf innerhalb einer vorgegebenen Zeitspanne T einen vorgegebenen Schwellwert a, wird steuerungsseitig davon ausgegangen, dass ein Stolpern vorliegt, beispielsweise dass der Fuß beim Gehen hängen bleibt oder mit der Ferse gegen ein Hindernis stößt, um dann eine Veränderung der Dämpfung vorzunehmen.

Üblicherweise wird die Dämpfung erhöht, das Niveau der Erhöhung kann variieren, üblicherweise wird das Niveau der Standphasendämpfung höher eingestellt, dies ist jedoch nicht zwingend notwendig.

Um zu entscheiden, ob eine Verringerung oder eine Erhöhung des Flexionswiderstandes stattfinden, kann eine Belastung des Unterteils 2 beispielsweise durch ein Auftreten eines Knöchelmomentes AM berücksichtigt werden. Tritt eine Axialkraft AF während einer normalerweise auftretenden Zeitspanne während des Gehens auf, kann mit einem Abbruch der Schwungphase und damit einer Störung des Gangmusters gerechnet werden, so dass eine Erhöhung oder Verringerung der Flexionsdämpfung angezeigt ist. Tritt eine Axialkraftkomponente in distaler Richtung auf, kann ein weiteres Durchschwingen des Prothesenfußes notwendig sein, so dass eine Verringerung des Flexionswiderstandes vorteilhaft ist und entsprechend eingestellt wird.

Neben dem Kniewinkel KA und dessen Ableitungen nach der Zeit kann auch die Belastung auf das Unterteil während der Schwungphase, also eine Axialkraft AF oder ein Knöchelmoment AM zur Steuerung herangezogen werden. Nach Erreichen eines Minimums nach dem toe-off, mit einer Axialkraft AF oder einem Knöchelmoment AM von Null, wird jedes Ansteigen der Axialkraft AF oder des Knöchelmomentes AM innerhalb einer normalerweise vorgesehenen Zeitspanne bis zum Heel Strike HS als Störung erkannt, die zur Veränderung der Dämpfereinstellung führt.

## Patentansprüche

1. Nicht-therapeutisches Verfahren zur Steuerung eines künstlichen Kniegelenkes mit einem Oberteil (1) und einem Unterteil (2), die schwenkbar um eine Schwenkachse (4) aneinander befestigt sind, und einer Widerstandseinheit (6), die zwischen dem Oberteil (1) und dem Unterteil (2) angeordnet ist und eine Verstelleinrichtung (7) aufweist, über die der Dämpfungswiderstand verändert werden kann, mit einer Steuereinheit (8), die mit der Verstelleinrichtung (7) gekoppelt ist und die zumindest mit einem Sensor (9) verbunden ist, wobei die Verstellung auf der Grundlage von Sensordaten erfolgt und während der Schwungphase zumindest eine Kenngröße aus einer Gruppe enthaltend den Kniewinkel (KA), die Kniewinkelgeschwindigkeit (KAV), die Kniewinkelbeschleunigung (KAA), den Unterschenkelwinkel, die Unterschenkelgeschwindigkeit, die Unterschenkelbeschleunigung, das Knöchelmoment (AM) und die Axialbelastung (AF) erfasst wird, **dadurch gekennzeichnet, dass** der Verlauf der Kenngröße bestimmt und der Dämpfungswiderstand verändert wird, wenn nach Erreichen eines Extremwertes der Kenngröße sich das Monotonieverhalten des Verlaufes der Kenngröße innerhalb der Schwungphase ändert.

2. Verfahren nach Anspruch 1, **dadurch gekennzeichnet, dass** der Dämpfungswiderstand erhöht wird.

3. Verfahren nach Anspruch 2, **dadurch gekennzeichnet, dass** der Dämpfungswiderstand auf ein Niveau der Standphasendämpfung oder darüber hinaus erhöht wird.

4. Verfahren nach einem der voranstehenden Ansprüche, **dadurch gekennzeichnet, dass** als Extremwert für den Unterschenkelwinkel, die Unterschenkelgeschwindigkeit, die Unterschenkelbeschleunigung, den Kniewinkel (KA), die Kniewinkelgeschwindigkeit (KAV) oder die Kniewinkelbeschleunigung (KAA) ein Maximum verwendet wird.

5. Verfahren nach einem der voranstehenden Ansprüche, **dadurch gekennzeichnet, dass** der Dämpfungswiderstand in der Schwungphase auf ein Niveau unterhalb eines Schwungphasenstandardwiderstandes abgesenkt wird, wenn das Knöchelmoment (AM) oder die Axialkraft (AF) Null sind oder einen Schwellwert unterschreiten und der Kniewinkel (KA), die Kniewinkelgeschwindigkeit (KAV) und/oder die Kniewinkelbeschleunigung (KAA) nach Erreichen eines Extremwertes eine Veränderung des Monotonieverhaltens erfährt.

6. Verfahren nach einem der voranstehenden Ansprüche, **dadurch gekennzeichnet, dass** für die Kenngrößen ein Schwellwert (a) festgelegt ist, der überschritten werden muss, damit der Dämpfungswiderstand verändert wird.

7. Verfahren nach einem der voranstehenden Ansprüche, **dadurch gekennzeichnet, dass** ein Zeitschwellwert (T) festgelegt ist, der nach dem Erreichen eines Extremwertes überschritten werden muss, damit der Dämpfungswiderstand verändert wird.

8. Verfahren nach einem der voranstehenden Ansprüche, **dadurch gekennzeichnet, dass** die Extremwerte der Kenngrößen in Echtzeit ermittelt werden.

9. Verfahren nach einem der voranstehenden Ansprüche, **dadurch gekennzeichnet, dass** die Veränderungen des Dämpfungswiderstandes registriert und in einem Speicher abgelegt werden.

10. Verfahren nach einem der voranstehenden Ansprüche, **dadurch gekennzeichnet, dass** diejenigen Monotonieänderungen nicht berücksichtigt werden, die in einem ungestörten Schwungphasenverlauf auftreten.

## Claims

1. A non-therapeutic method for controlling an artificial knee joint having an upper part (1) and a lower part (2) which are fastened to one another in a manner pivotable about a pivot axis (4) and having a resistance unit (6) which is arranged between the upper part (1) and the lower part (2) and which has an adjustment device (7) by means of which the damping resistance can be varied, having a control unit (8) which is coupled to the adjustment device (7) and which is connected at least to a sensor (9), wherein the adjustment is carried out on the basis of sensor data and during the swing phase, at least one characteristic from the group containing the knee angle (KA), the knee angle velocity (KAV), the knee angle acceleration (KAA), the below-knee angle, the below-knee velocity, the below-knee acceleration, the ankle moment (AM) and the axial load (AF) is captured, **characterized in that** the profile of the characteristic is determined and the damping resistance is varied if, after reaching an extremum of the characteristic, the monotonic behavior of the profile of the characteristic changes within the swing phase.

2. The method as claimed in claim 1, **characterized in that** the damping resistance is increased.

3. The method as claimed in claim 2, **characterized in that** the damping resistance is increased to a level of the stance phase damping or therebeyond.

4. The method as claimed in any one of the preceding claims, **characterized in that** a maximum is used as an extremum for the below-knee angle, the below-knee velocity, the below-knee acceleration, the knee angle (KA), the knee angle velocity (KAV) or the knee angle acceleration (KAA).

5. The method as claimed in any one of the preceding claims, **characterized in that** the damping resistance in the swing phase is lowered to a level below a swing phase standard resistance if the ankle moment (AM) or the axial force (AF) are zero or have dropped below a threshold and the knee angle (KA), the knee angle velocity (KAV) and/or the knee angle acceleration (KAA) experiences a change in the monotonic behavior after reaching an extremum.

6. The method as claimed in any one of the preceding claims, **characterized in that** a threshold (a) that needs to be exceeded so that the damping resistance is varied is set for the characteristics.

7. The method as claimed in any one of the preceding claims, **characterized in that** a time threshold (T) that needs to be exceeded after reaching an extremum so that the damping resistance is varied is set.

8. The method as claimed in any one of the preceding claims, **characterized in that** the extrema of the characteristics are ascertained in real time.

9. The method as claimed in any one of the preceding claims, **characterized in that** the variations of the damping resistance are registered and stored in a memory.

10. The method as claimed in any one of the preceding claims, **characterized in that** those changes in the monotonic behavior that occur during the course of an unimpeded swing phase are not taken into account.

## Revendications

1. Procédé non thérapeutique pour commander une articulation de genou artificielle, comportant une partie supérieure (1) et une partie inférieure (2) qui sont fixées l'une à l'autre de façon mobile en pivotement autour d'un axe de pivotement (4), et une unité de résistance (6) qui est agencée entre la partie supérieure (1) et la partie inférieure (2) et qui présente un moyen de réglage (7) permettant de varier la résistance d'amortissement, comportant une unité de commande (8) couplée au moyen de réglage (7) et connectée à au moins un capteur (9), le réglage s'effectuant sur la base de données de capteur et, pendant la phase pendulaire, au moins une grandeur caractéristique est détectée parmi un groupe comprenant l'angle du genou (KA), la vitesse angulaire du genou (KAV), l'accélération angulaire du genou (KAA), l'angle du bas de jambe, la vitesse du bas de jambe, l'accélération du bas de jambe, le couple de cheville (AM) et la charge axiale (AF),
**caractérisé en ce que**
l'évolution de la grandeur caractéristique est déterminée et la résistance d'amortissement est variée lorsque, une fois qu'une valeur extrême de la grandeur caractéristique est atteinte, le comportement de monotonie de l'évolution de la grandeur caractéristique varie à l'intérieur de la phase pendulaire.

2. Procédé selon la revendication 1, **caractérisé en ce que** la résistance d'amortissement est augmentée.

3. Procédé selon la revendication 2, **caractérisé en ce que** la résistance d'amortissement est augmentée à un niveau de l'amortissement de phase debout ou au-delà de celui-ci.

4. Procédé selon l'une des revendications précédentes, **caractérisé en ce qu'**un maximum est utilisé à titre de valeur extrême pour l'angle du bas de jambe, la vitesse du bas de jambe, l'accélération du bas de jambe, l'angle du genou (KA), la vitesse angulaire du genou (KAV) ou l'accélération angulaire du genou (KAA).

5. Procédé selon l'une des revendications précédentes, **caractérisé en ce que** la résistance d'amortissement dans la phase pendulaire est abaissée à un niveau au-dessous de la résistance standard de phase pendulaire lorsque le couple de cheville (AM) ou la force axiale (AF) sont zéro ou passent au-dessous d'une valeur seuil, et l'angle du genou (KA), la vitesse angulaire du genou (KAV) et/ou l'accélération angulaire du genou (KAA) subit une variation du comportement de monotonie, une fois qu'une valeur extrême est atteinte.

6. Procédé selon l'une des revendications précédentes, **caractérisé en ce qu'**une valeur seuil (a) est fixée pour les grandeurs caractéristiques, qui doit être dépassée pour que la résistance d'amortissement soit variée.

7. Procédé selon l'une des revendications précédentes, **caractérisé en ce qu'**une valeur seuil temporelle (T) est fixée qui doit être dépassée, une fois qu'une valeur extrême est atteinte, pour que la résistance d'amortissement soit variée.

8. Procédé selon l'une des revendications précédentes, **caractérisé en ce que** les valeurs extrêmes des grandeurs caractéristiques sont déterminées en temps réel.

9. Procédé selon l'une des revendications précédentes, **caractérisé en ce que** les variations de la résistance d'amortissement sont enregistrées et stockées dans une mémoire.

10. Procédé selon l'une des revendications précédentes, **caractérisé en ce que** celles des variations de monotonie qui apparaissent pendant une évolution non perturbée de la phase pendulaire ne sont pas prises en compte.
